(19) Europäisches Patentamt
European Patent Office
Office européen des brevets

(11) Publication number: **0 307 221 B1**

(12) **EUROPEAN PATENT SPECIFICATION**

(45) Date of publication of patent specification: **02.06.93**

(51) Int. Cl.5: **C07D 493/22**, //(C07D493/22, 313:00,311:00,307:00,307:00)

(21) Application number: **88308345.3**

(22) Date of filing: **09.09.88**

(54) **Process for the preparation of macrolide compounds.**

(30) Priority: **11.09.87 GB 8721373**

(43) Date of publication of application:
**15.03.89 Bulletin 89/11**

(45) Publication of the grant of the patent:
**02.06.93 Bulletin 93/22**

(84) Designated Contracting States:
**AT BE CH DE ES FR GB GR IT LI LU NL SE**

(56) References cited:
**EP-A- 0 262 384**
**GB-A- 2 176 182**
**US-A- 4 429 209**

**HOUBEN-WEYL: "Methoden der organischen Chemie", vol. IV/1c, edition 4, 1980, pages 714-717, Georg Thieme Verlag, Stuttgart, DE**

(73) Proprietor: **AMERICAN CYANAMID COMPANY**
**One Cyanamid Plaza**
**Wayne New Jersey 07470(US)**

(72) Inventor: **Ramsay, Michael V. J.**
**157 Kings Road**
**South Harrow Middlesex(GB)**
Inventor: **Freeman, Stephen**
**2 Coronation Cottages Downley Common**
**Downley**
**High Wycombe Buckinghamshire(GB)**
Inventor: **Shingler, Anthony H.**
**21 Western Gardens**
**Ealing London(GB)**
Inventor: **Pereira, Oswy Z.**
**32 Old Park Mews Heston**
**Hounslow Middlesex(GB)**
Inventor: **Dolan, Simon C.**
**387 Torbay Road**
**Harrow Middlesex(GB)**

(74) Representative: **Skailes, Humphrey John et al**
**Frank B. Dehn & Co. Imperial House 15-19**
**Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to a novel process for the preparation of macrolide compounds.

The compounds of formula (I)

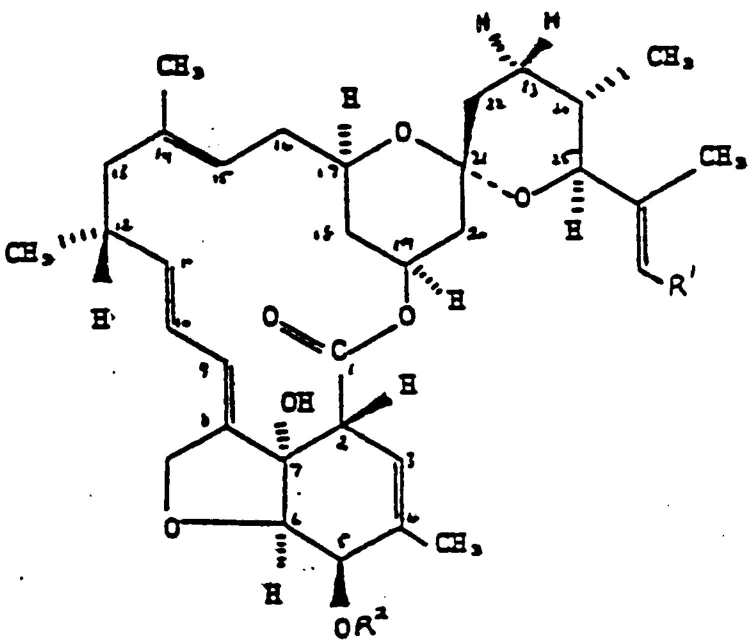

(I)

wherein $R^1$ represents a methyl, ethyl or isopropyl group and $OR^2$ represents a hydroxy, methoxy or acetyloxy group) are described in UK Patent Specification 2176182A. These compounds have anti-endoparasitic, anti-ectoparasitic, anti-fungal, insecticidal, nematicidal and acaricidal activity and are useful in combating parasites in animals and humans and pests in agriculture, horticulture, forestry, public health and stored products. The compounds may also be used as intermediates in the preparation of other active compounds.

The present invention provides a novel and useful multi-step synthesis of the compounds of formula (I). In particular, we describe the conversion of a 23-hydroxy compound into a 23-halo compound with inversion of configuration followed by reduction to give the desired 23-desoxy compounds of formula (I). The overall process is convenient to use and provides the compounds of formula (I) in good yield. Furthermore, the reaction can, if desired, be carried out without isolating the intermediates of formulae (II) or (III).

Thus, in one aspect of the present invention, we provide a process for preparing a compound of formula (I) which comprises reducing a compound of formula (II)

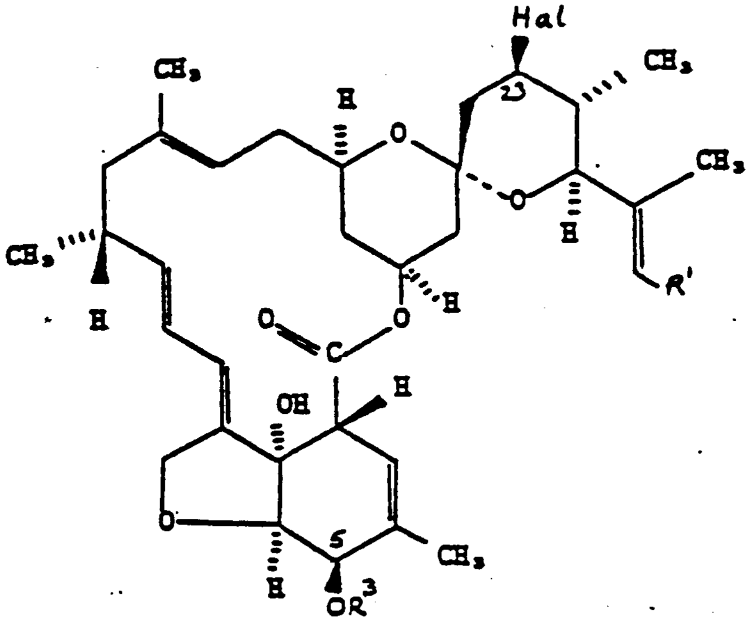

(II)

(where $R^1$ is as defined above, $R^3$ is a hydrogen atom, a methyl group or a protecting group and Hal represents a halogen atom e.g. chlorine, bromine or iodine) followed, if desired, by acetylation of a compound of formula (I) in which $OR^2$ is a hydroxyl group to form a compound of formula (I) in which $OR^2$ is an acetyloxy group or by deprotection to form a 5-hydroxy compound of formula (I).

The reduction may be effected with a reducing agent which is capable of replacing the halogen atom in the compound of formula (II) with a hydrogen atom. Suitable reducing agents include zinc metal or a zinc-copper couple, in the presence of a base or a proton source e.g. ammonium chloride or glacial acetic acid. The reaction takes place in a solvent such as an alkanol (e.g. methanol, isopropyl alcohol or t-butyl alcohol), a sulphoxide (e.g. dimethylsulphoxide or a nitrile (e.g. acetonitrile) at an elevated temperature e.g. reflux.

Examples of suitable protecting groups for $R^3$ are described in "Protective Groups in Organic Synthesis" by Theodora W. Greene (Wiley-Interscience, New York 1981) and "Protective Group in Organic Chemistry" by J.F.W. McOmie (Plenum Press, London, 1973). Such groups may be introduced and removed by conventional methods.

Thus, for example, an acyl group such as an acetyl group may be removed by basic hydrolysis e.g. using sodium or potassium hydroxide in aqueous alcohol or using potassium carbonate in an alcohol such as methanol or by acid hydrolysis e.g. using concentrated sulphuric acid in methanol. Acetal groups such as tetrahydropyranyl may be removed, for example, using acid hydrolysis (using an acid such as acetic or trifluoroacetic acid or a dilute mineral acid). Silyl groups may be removed using fluoride ions (e.g. from a tetraalkylammonium fluoride such as tetra-n-butylammonium fluoride), hydrogen fluoride in aqueous acetonitrile or an acid such as p-toluene sulphonic acid (e.g. in methanol). Arylmethyl groups may be removed by treatment with a Lewis acid (e.g. boron trifluoride-etherate) in the presence of a thiol (e.g. ethanethiol) in a suitable solvent such as dichloromethane at e.g. room temperature.

A particularly useful protecting group for $R^3$ is trichloroethoxycarbonyl which may be cleaved under the reducing conditions previously described.

The acetylation reaction may be carried out using standard procedures.

Thus, for example, acetylation may be affected using an acetylating agent such as acetic acid or a reactive derivative thereof, such as an acetyl halide (e.g. acetyl chloride), anhydride or activated ester, or a reactive derivative or a carbonic acid $CH_3OCOOH$ or thiocarbonic acid $CH_3OCSOH$.

Acetylations employing acetyl halides and anhydrides may if desired be effected in the presence of an acid binding agent such as a tertiary amine (e.g. triethylamine, dimethylaniline or pyridine), inorganic bases (e.g. calcium carbonate or sodium bicarbonate), and oxiranes such as lower 1,2-alkylene oxides (e.g. ethylene oxide or propylene oxide) which bind hydrogen halide liberated in the acetylation reaction.

Acetylation employing acetic acid is desirably conducted in the presence of a condensing agent, for example a carbodiimide such as N,N′-dicyclohexylcarbodiimide or N-ethyl-N′γ-dimethylaminopropylcarbodiimide; a carbonyl compound such as carbonyldiimidazole; or an isoxazolium salt such as N-ethyl-5-phenylisoxazolium perchlorate.

An activated ester may conveniently be formed in situ using, for example, 1-hydroxybenzotriazole in the presence of a condensing agent as set out above. Alternatively, the activated ester may be preformed.

The acetylation reaction may be effected in aqueous or non-aqueous reaction media, conveniently at a temperature in the range -20° to +100°C, e.g. -10° to +50°C.

Particularly useful intermediates of formula (II) are those in which $R^1$ represents an isopropyl group, Hal is a bromine atom and $R^3$ is as defined above, and more especially a hydrogen atom, or an acetyl or trichloroethoxycarbonyl group.

The compounds of formula (II) may be prepared by treating a compound of formula (III)

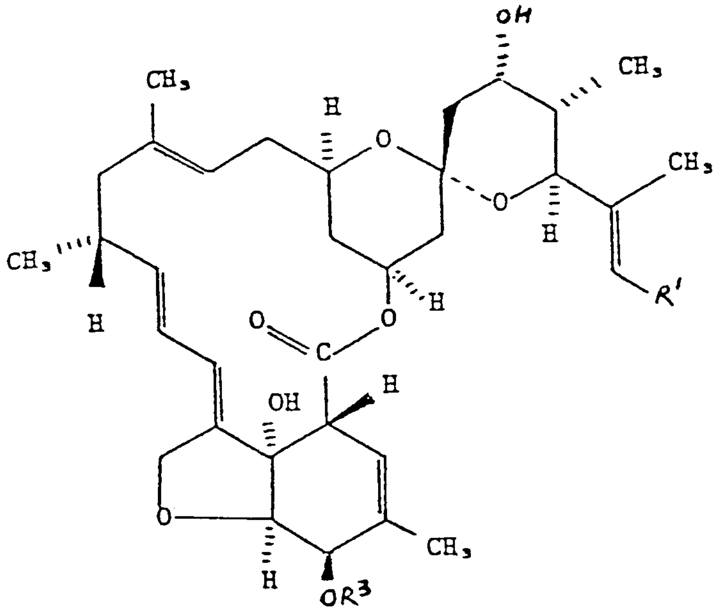

(III)

[wherein $R^1$ is as defined in formula (I) and $R^3$ is as defined in formula (II)] with a suitable halogenating agent.

The reaction may be effected with a halogenating agent serving to replace the 23-OH group by a halogen atom with inversion of configuration, whereby a compound of formula (II) is produced.

Bromination may be effected with one of the following systems: a triarylphosphine (e.g. triphenylphosphine) and bromine in dimethylformamide; phosphorus tribromide in dimethylformamide; trialkylsilyl bromides such as trimethylsilyl bromide; and lithium bromide and trimethylsilyl chloride.

Iodination may conveniently be effected with sodium iodide and a trialkylsilyl chloride e.g. trimethylsilyl chloride.

Chlorination may be effected using, for example, a trialkylsilyl chloride (e.g. trimethylsilyl chloride).

The reaction is conveniently carried out in a solvent e.g. a nitrile such as acetonitrile, an ester (e.g. ethyl acetate) or a halogenated hydrocarbon (e.g. methylene chloride) at a temperature in the range of 0° to 50°C, conveniently at 20-30°C.

Compounds of formula (II) in which $OR^2$ represents a hydroxyl group may also be prepared from a compound of formula (IV)

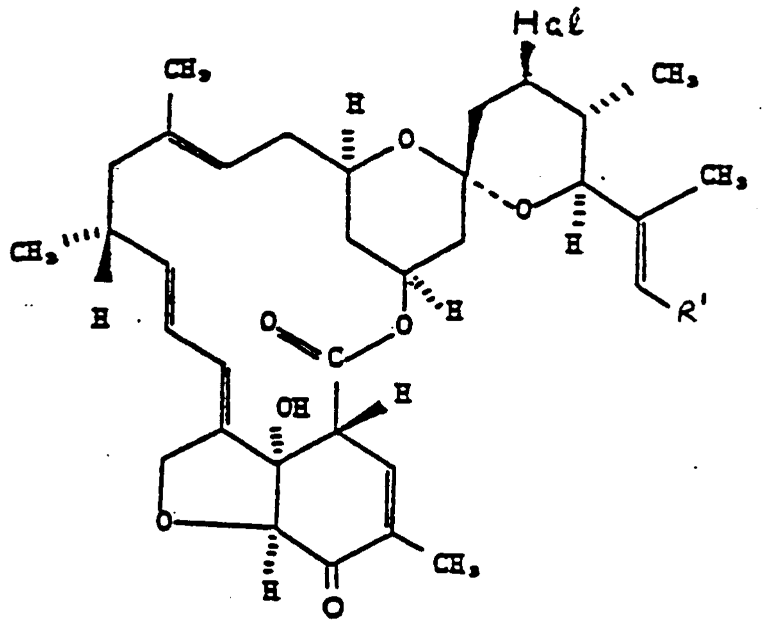

(IV)

wherein $R^1$ is as defined in formula (I) and Hal represents a halogen atom as defined previously.

Thus, in further aspect of the present invention, we provide a process for preparing a compound of formula (II) in which $OR^2$ is a hydroxyl group which comprises reducing a compound of formula (IV).

The reduction may be effected with a reducing agent which is capable of stereoselectively reducing the 5-keto group. Suitable reducing agents include borohydrides such as alkali metal borohydrides (e.g. sodium borohydride) and lithium alkoxyaluminium hydrides such as lithium tributoxyaluminium hydride.

The reaction involving a borohydride reducing agent takes place in the presence of a solvent such as an alkanol e.g. isopropyl alcohol or isobutyl alcohol conveniently at a temperature in the range of -30° to +80°C e.g. at 0°C. The reaction involving a lithium alkoxyaluminium hydride takes place in the presence of a solvent such as an ether e.g. tetrahydrofuran or dioxan conveniently at a temperature in the range of -78° to 0°C e.g. at -78°C.

The compounds of formula (IV) may be prepared by treating a compound of formula (V)

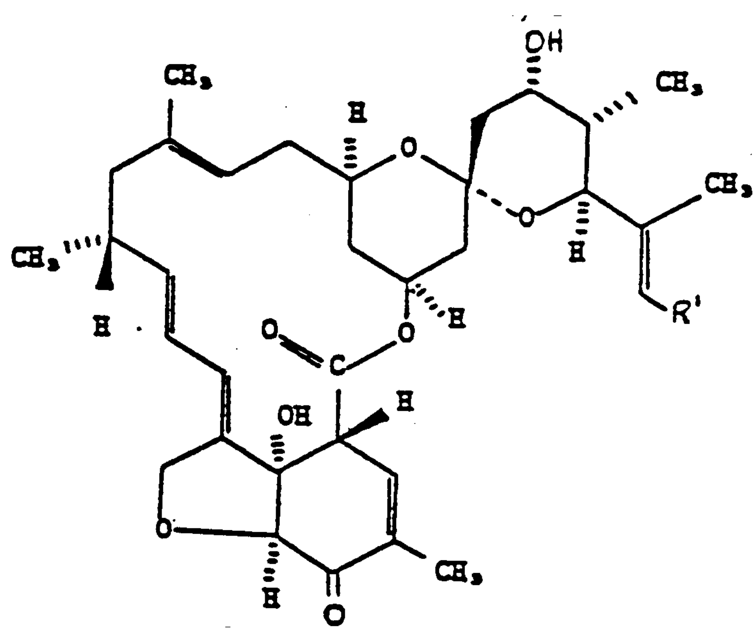

(V)

[wherein $R^1$ is as defined in formula (I)] with a suitable halogenating agent according to the method described above for the preparation of compounds of formula (II) from compounds of formula (III).

The compounds of formula (III) in which $R^2$ represents a hydrogen atom or a methyl group may be obtained using the fermentation and isolation methods described in UK Patent Specification No. 2166436A. The compounds of formula (III) in which $R^2$ represents an acetyl group may be prepared from the corresponding 5-OH compounds using the standard acetylation procedures described above.

Compounds of formula (V) and their preparation are described in EP-A 0238258 and GB-A 2187742.

A particular embodiment of the present process involves the preparation of a compound of formula (I) in which $R^1$ is an isopropyl group and $R^2$ is a hydrogen atom.

The invention is illustrated but not limited by the following Examples in which temperatures are in °C and 'L' represents litre.

The compound of formula (V) in which $R^1$ is an isopropyl group which is referred to hereinafter as 'Factor I' and its preparation is decsribed in GB-A 2187742.

The intermediate of formula (III) in which $R^1$ is an isopropyl group and $OR^2$ is a hydroxyl group is hereinafter referred to as 'Factor A'.

Preparation 1

Factor A 5-Acetate

Factor A (3.0g) in pyridine (20 ml) at -5° was treated with acetic anhydride (8 ml) and the resulting solution left at 3° for 20 hr. Benzene (100 ml) was added and the solution concentrated in vacuo. The residual oil was chromatographed over silica using dichloromethane:acetone (40:1) as eluent to give Factor A 5-acetate (2.06 g), containing 10% Factor A 5,23-diacetate. The compounds were separated by reverse-phase preparative hplc to give the title compound (79% recovery), $\lambda_{max}$ (EtOH) 244.5 nm ($E_1^1$ 462), $\delta$ ($CDCl_3$) includes 2.14 (s; 3H), m/z includes 654, 594 and 576.

Example 1

23-Desoxy Factor A

(i) 23-Desoxy-23-epi-bromo Factor I

(a) A stirred suspension of Factor I (500mg) in acetonitrile (10ml) was warmed to 30° and bromotrimethylsilane (0.13ml) was added. After 30 minutes the mixture was partitioned between ethyl acetate and 2N hydrochloric acid. The organic phase was separated and washed successively with 2N hydrochloric acid and saturated sodium bicarbonate solution and then dried over sodium sulphate and the solvent was evaporated. The residue was chromatographed over a column of silica (Merck Art 9385; 150ml) made up in hexane (60-80°)/ethyl acetate (4:1) and eluted with the same solvent. Appropriate fractions of the major component were combined and the solvent was evaporated to give the title compound as a foam (320mg) $[\alpha]_D^{22}$ + 4.3° (c 0.46; $CHCl_3$);

$$\lambda_{max}^{EtOH} \text{ 240nm } (\varepsilon \text{ 26,000});$$

$\nu_{max}$ ($CHBr_3$) 3500 (OH), 1710 (ester), 1678 (ketone), 997 (C-O); $\delta$ ($CDCl_3$) include 0.95 (d;3H), 0.97 (d;3H), 1.00 (d;3H), 3.58 (m,1H), 4.19 (t;d;1H), 3.83 (s;1H), 3.85 (s;1H), and 6.58 (m;1H).

(b) Bromine (0.15ml) was added dropwise to a stirred solution of triphenylphosphine (0.74g) in dimethylformamide (2.6ml) under nitrogen. The suspension was allowed to cool to room temperature and was diluted with ether. The solvent was decanted off and the solid was washed with ether. The dried solid was redissolved in acetonitrile (20ml) and an aliquot (5ml) was cooled in an ice bath and a solution of 5-keto Factor A (100mg) in acetonitrile (2ml) was added under nitrogen. After 1h the solution was allowed to warm to room temperature and the reaction was left for 20h. The mixture was partitioned between ethyl acetate and 2N hydrochloric acid and the organic phase was separated. The organic phase was washed with 2N hydrochloric acid, then with saturated sodium bicarbonate solution, then was dried over sodium sulphate, and the solvent was evaporated. The residue was chromatographed over a column of silica (Merck Art 9385; 75ml) made up in hexane (60-80°)/ethyl acetate and eluted with the same solvent. Appropriate fractions of the major component were combined and the solvent was evaporated to leave the title compound as a foam (20mg).

(ii) 23-Desoxy-23-epi-bromo Factor A

(a) A stirred solution of 23-desoxy-23-epi-bromo Factor I (50mg) in dry isopropanol (2ml) was cooled to 0° under nitrogen and sodium borohydride (3mg) was added. After 30 minutes the mixture was diluted with ether and 2N hydrochloric acid was added dropwise. The organic phase was separated and was washed with 2N hydrochloric acid, dried over sodium sulphate and the solvent evaporated. The residue was chromatographed over a column of silica (Merck Art 9385; 50ml) made up in hexane (60-80°)/ethyl acetate (2:1) and eluted with the same solvent. Appropriate fractions of the major component were combined and the solvent was evaporated to leave the title compound as a foam (26mg). $[\alpha]_D^{22}$ + 71° (c 0.29; $CHCl_3$);

$$\lambda_{max}^{EtOH}\ 244.4nm\ (\epsilon\ 27,840);$$

$\nu_{max}$ ($CHBr_3$) 3550, 3470 (OH), 1706 (ester), 993 (C-O); $\delta$ ($CDCl_3$) values include 0.93 (d;3H), 0.95 (d;3H), 1.00 (d;3H), 3.28 (m;1H), 3.90 (s;1H), 3.95 (s;1H), 4.19 (t;d;1H), 4.29 (t;1H), 5.42 (s;1H).

(b) 23-Desoxy-23-epi-bromo Factor A

A stirred solution of 23-desoxy-23-epi-bromo Factor I (50mg) in dry tetrahydrofuran (5ml) under nitrogen was cooled to ca -70°. Lithium tri-t-butoxyaluminohydride (110mg) was added and after 1h an additional portion of lithium tri-t-butoxyaluminohydride (95mg) was added. After a further 1.5h the reaction mixture was diluted with ethyl acetate and then aqueous 2N hydrochloric acid was added slowly. The organic phase was separated and was washed successively with 2N hydrochloric acid and saturated sodium bicarbonate solution and then dried over sodium sulphate. The solvent was evaporated to leave the title compound as a foam (53mg).

(iii) 23-Desoxy Factor A

A solution of 23-desoxy-23-epi-bromo Factor A (350mg) in isopropyl alcohol (15ml) was treated with glacial acetic acid (1.5ml), then with zinc dust (350mg). The stirred mixture was heated under reflux for 6h, allowed to cool and then filtered. The solution was diluted with ethyl acetate, washed successively with 2N hydrochloric acid and saturated sodium bicarbonate solution and the organic solution was dried over sodium sulphate and the solvent was evaporated. The residue was chromatographed over a column of silica (Merck Art 9385; 100ml) made up in hexane (60-80°)/ethyl acetate (3:1) and eluted with the same solvent. Appropriate fractions of the major component were combined and the solvent was evaporated to give a foam (205mg). This material was rechromatographed over a column of silica (Merck Art 9385; 150ml) made up in dichloromethane/ether (9:1) and eluted with the same solvent. Appropriate fractions of the major component were combined and the solvent was evaporated to give the title compound as a foam (100mg), $\delta$ ($CDCl_3$) include 3.27 (m;1H), 3.42 (d9; 1H), 3.54 (m, 1H) and 4.29 (t6; 1H), m/z include 596, 578, 560, 468, 450, 356, 314, 299, 249, 248, 221 and 151.

Example 2

23-Desoxy Factor A 5-acetate

Chlorotrimethylsilane (580 mg) was added to a stirred solution of sodium iodide (570 mg) is acetonitrile (10 ml), under nitrogen. The solution was stirred at room temperature for 20 minutes, then added to a stirred solution of Factor A 5-acetate (1.0 g) in acetonitrile (10 ml), heated under reflux. The mixture was treated with glacial acetic acid (1.0 ml), cooled to room temperature, and zinc dust (1.0 g) added. After a 2 hour reflux the stirred suspension was allowed to cool, and then filtered. The filtrate was diluted with ethyl acetate, washed with saturated sodium chloride solution, and the solvent evaporated to give the title compound as a foam (1.0 g), $[\alpha]_D^{22}$ + 144° (c. 0.43 chloroform), $\lambda_{max}$ (ethanol) 245.5nm ($\epsilon$ 29650), $\nu_{max}$ - ($CHBr_3$) 3420-3340 (OH), 1732 (acetate), 1710cm$^{-1}$ (carbonyl), $\delta$ ($CDCl_3$) include 0.68 (d,5Hz,3H), 2.16 (s,3H), 3.32 (m,1H).

Example 3

23-Desoxy Factor A

(i) Factor A 5-trichloroethoxycarbonate

To a stirred solution of Factor A (10 g) in dichloromethane (100 ml) cooled to 5 °C, was added pyridine (5 ml) and a solution of trichloroethylchloroformate (2.7 ml) in dichloromethane (10 ml) dropwise. After 45 minutes the mixture was washed with 2N hydrochloric acid, then with saturated sodium bicarbonate solution and saturated sodium chloride solution and the solvent evaporated to give the title compound as a foam (12.05 g), $\nu_{max}$ ($CHBr_3$) 3500 (OH), 1755 (carbonate), 1710 (ester), 1242 (C-O); $\delta$ ($CDCl_3$) include 0.80 (d; 3H), 0.96 (d; 3H), 1.06 (d; 3H), 3.35 (m; 1H), 4.13 (d; 1H), 4.75 and 4.90 (each d; 2H), and 5.6 (brs, 1H).

(ii) 23-Desoxy-23-epi-bromo Factor A 5-trichloroethoxycarbonate

A stirred solution of Factor A 5-trichloroethoxycarbonate (500 mg) in acetonitrile (15 ml) was warmed to 30 °C and bromotrimethylsilane (0.1 ml) was added. After 30 minutes the mixture was partitioned between ethyl acetate and 2N hydrochloric acid, then the ethyl acetate layer dried over sodium sulphate and the solvent evaporated. The residue was chromatographed over a column of silica (Merck Art 9385; 150 ml) made up in dichloromethane/ethyl acetate (15:1) and eluted with the same solvent. Appropriate fractions were combined and the solvent was evaporated to give the title compound as a foam (350 mg) $\nu_{max}$ ($CHBr_3$) 3450 (OH), 1760 (carbonate), 1708 (lactone); $\delta$ ($CDCl_3$) include 0.93 (d; 3H), 0.95 (d; 3H), 1.00 (d; 3H), 3.36 (m; 2H) 3.48 (m; 1H), 4.20 (m; 1H), 4.75 and 4.89 (each d; 2H) and 5.61 (brs, 1H).

(iii) 23-Desoxy Factor A

A solution of 23-desoxy-23-epi-bromo Factor A 5-trichloroethoxycarbonate (350 mg) in isopropyl alcohol (20 ml) was treated with glacial acetic acid (1.5 ml), then zinc dust (350 mg). The stirred mixture was heated under reflux for 6h, allowed to cool and then filtered. The solution was diluted with ethyl acetate, washed successively with 2N hydrochloric acid, saturated sodium bicarbonate solution and the organic solution dried over sodium sulphate and the solvent evaporated. The residue was chromatographed over a column of silica (Merck Art 9385; 100 ml) made up in dichloromethane/ethyl acetate (15:1) and eluted with the same solvent. Appropriate fractions of the major component were combined and the solvent evaporated to give the title compound as a foam (180 mg). NMR and m/z values correspond with those of the product of Example 1.

Example 4

23-Desoxy-Factor A

(i) 23-Desoxy-23-epi-bromo Factor A

A stirred solution of Factor A (2.0 g) in acetonitrile (25 ml) was warmed to 30 °C and bromotrimethylsilane (0.52 ml) was added. After 30 minutes the mixture was partitioned between ethyl acetate and 2N hydrochloric acid. The organic phase was separated and washed successively with 2N hydrochloric acid, saturated sodium bicarbonate and then dried over sodium sulphate and the solvent evaporated. The residue was chromatographed over a column of silica (Merck Art 9385; 200 ml) made up in dichloromethane and eluted with dichloromethane/ethyl acetate (15:1). Appropriate fractions of the major component were combined and the solvent was evaporated to give the title compound as a foam (553 mg). The spectral data corresponded with the data for the product of Example 1(ii).

(ii) 23-Desoxy Factor A

A solution of 23-desoxy-23-epi-bromo Factor A (100 mg) in isopropyl alcohol (10 ml) was treated with glacial acetic acid (1.0 ml), then with zinc dust (100 mg). The stirred mixture was heated under reflux for 6h, allowed to cool and then filtered. The solution was diluted with ethyl acetate, washed successively with 2N hydrochloric acid and saturated sodium bicarbonate solution and the organic solution was dried over sodium sulphate and the solvent was evaporated. The residue was chromatographed over a column of silica (Merck

Art 9385; 100 ml) made up in dichloromethane/ethyl acetate (15:1) and eluted with the same solvent. Appropriate fractions of the major component were combined to give the title compound as a foam (60 mg). NMR and m/z values corresponded with those of the product of Example 1.

## Claims

**1.** A process for preparing a compound of formula (I)

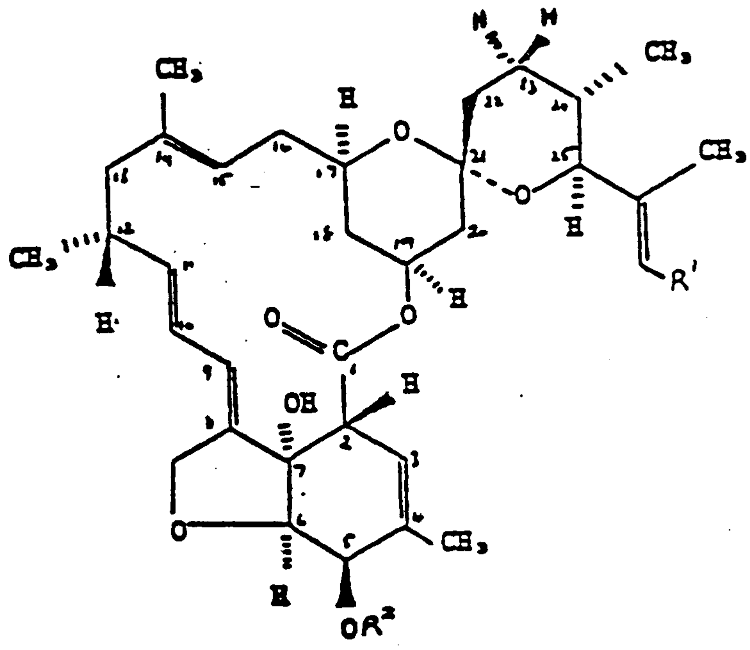

(I)

(wherein $R^1$ represents a methyl, ethyl or isopropyl group and $OR^2$ represents a hydroxy, methoxy or acetyloxy group) which comprises reducing a compound of formula (II)

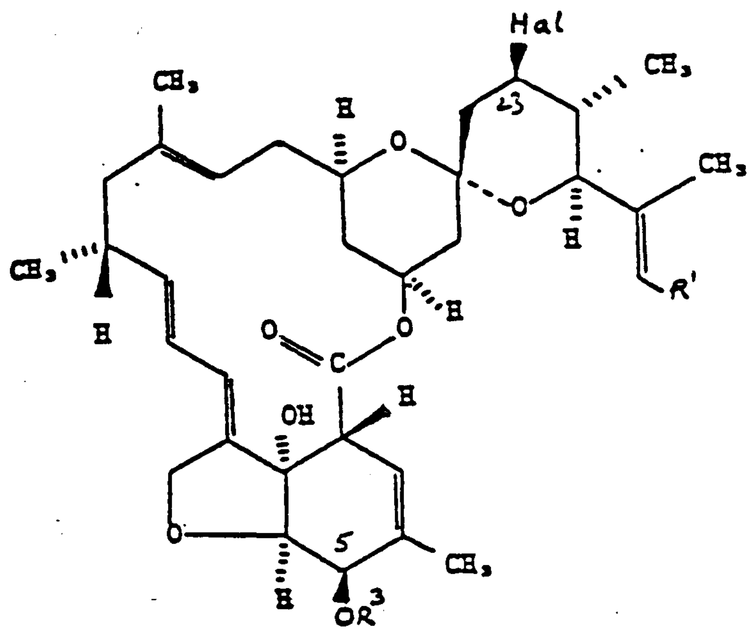

(II)

(wherein $R^1$ is as defined above, $R^3$ is a hydrogen atom, a methyl group or a protecting group and Hal represents a halogen atom) followed, if desired, by acetylation of a compound of formula (I) in which $OR^2$ is a hydroxyl group to form a compound of formula (I) in which $OR^2$ is an acetyloxy group or by

deprotection to form a 5-hydroxy compound of formula (I);

with the proviso that when $R^3$ is a hydrogen atom or a methyl group, the reduction is effected with zinc metal or a zinc-copper couple in the presence of a base or a proton source.

**2.** A process according to claim 1 in which the reduction is effected with zinc metal or a zinc-copper couple in the presence of a base or ammonium chloride or acetic acid.

**3.** A process according to claim 1 in which the compound of formula (II) is prepared by halogenating the corresponding 23α-hydroxy compound.

**4.** A process according to claim 3 in which a bromo compound of formula (II) is prepared by bromination of the corresponding 23α-hydroxy compound with triphenylphosphine and bromine in dimethylformamide; phosphorous tribromide in dimethylformamide; trimethylsilyl bromide; or lithium bromide and trimethylsilyl chloride.

**5.** A process according to claim 1 in which the compound of formula (II) is prepared by reducing the corresponding 5-keto compound.

**6.** A process according to claim 1 in which the compound of formula (II) is prepared by reducing the corresponding 5-keto compound with an alkali metal borohydride or a lithium alkoxyaluminium hydride.

**7.** A process according to claim 5 in which the said 5-keto compound is prepared by halogenation of the corresponding 5-keto 23α-hydroxy compound.

## Patentansprüche

**1.** Verfahren zur Herstellung einer Verbindung der Formel (I)

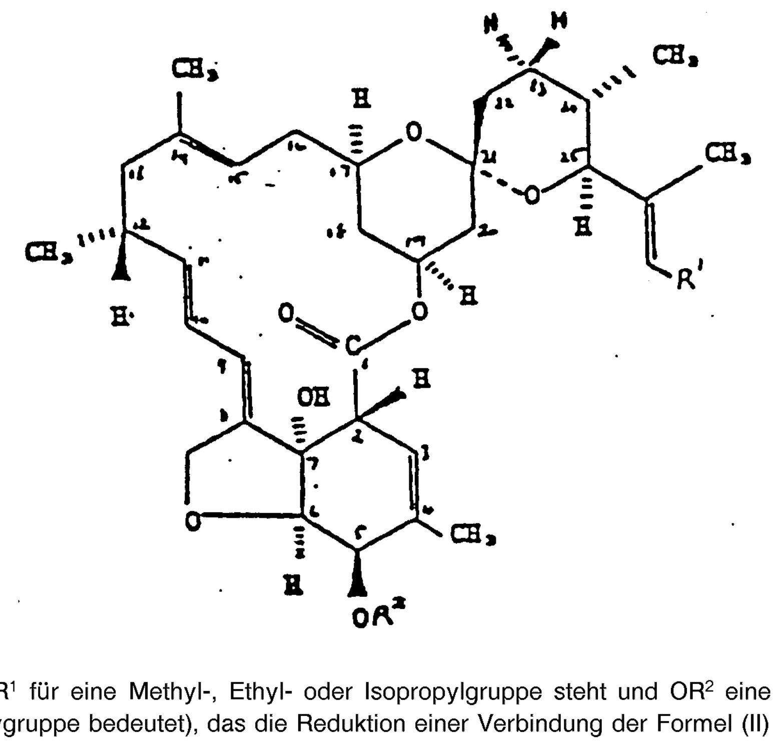

(I)

(worin $R^1$ für eine Methyl-, Ethyl- oder Isopropylgruppe steht und $OR^2$ eine Hydroxy-, Methoxy- oder Acetoxygruppe bedeutet), das die Reduktion einer Verbindung der Formel (II)

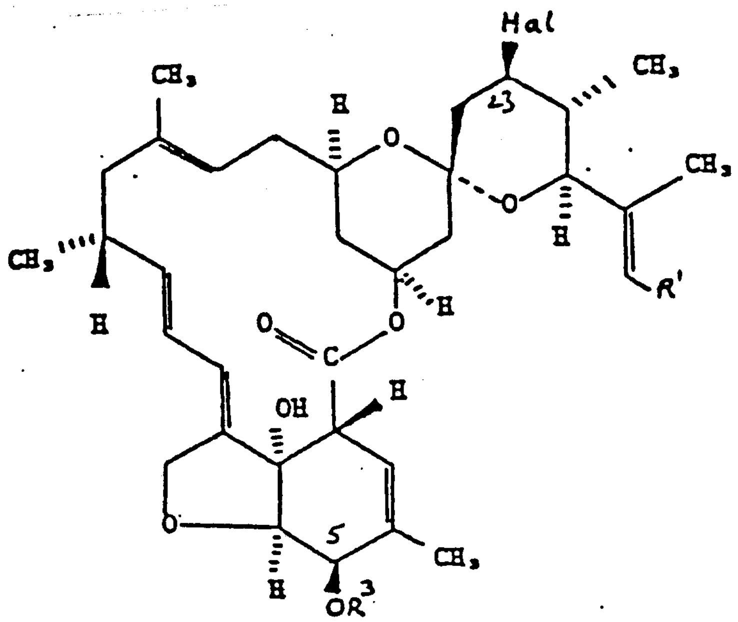

(II)

(worin $R^1$ wie vorstehend definiert ist, $R^3$ für ein Wasserstoffatom, eine Methylgruppe oder eine Schutzgruppe steht und Hal ein Halogenatom bedeutet) umfaßt, woran sich gegebenen falls eine Acetylierung einer Verbindung der Formel (I), worin $OR^2$ eine Hydroxygruppe bedeutet, zur Bildung einer Verbindung der Formel (I), worin $OR^2$ eine Acetoxygruppe bedeutet, oder eine Schutzgruppenabspaltung zur Bildung einer 5-Hydroxy-Verbindung der Formel (I) anschließt;
mit der Maßgabe, daß wenn $R^3$ für ein Wasserstoffatom oder eine Methylgruppe steht, die Reduktion mit metallischem Zink oder einem Zink-Kupfer-Paar in Gegenwart einer Base oder einer Protonenquelle durchgeführt wird.

2. Verfahren gemäß Anspruch 1, worin die Reduktion mit metallischem Zink oder einem Zink-Kupfer-Paar in Gegenwart einer Base oder von Ammoniumchlorid oder Essigsäure durchgeführt wird.

3. Verfahren gemäß Anspruch 1, worin die Verbindung der Formel (II) durch Halogenierung der entsprechenden 23α-Hydroxy-Verbindung hergestellt wird.

4. Verfahren gemäß Anspruch 3, worin eine Bromverbindung der Formel (II) durch Bromierung der entsprechenden 23α-Hydroxy-Verbindung mit Triphenylphosphin und Brom in Dimethylformamid; Phosphortribromid in Dimethylformamid; Trimethylsilylbromid; oder Lithiumbromid und Trimethylsilylchlorid hergestellt wird.

5. Verfahren gemäß Anspruch 1, worin die Verbindung der Formel (II) durch Reduktion der entsprechenden 5-Keto-Verbindung hergestellt wird.

6. Verfahren gemäß Anspruch 1, worin die Verbindung der Formel (II) durch Reduktion der entsprechenden 5-Keto-Verbindung mit einem Alkalimetallborhydrid oder einem Lithium-alkoxyaluminiumhydrid hergestellt wird.

7. Verfahren gemäß Anspruch 5, worin die 5-Keto-Verbindung durch Halogenierung der entsprechenden 5-Keto-23α-hydroxy-Verbindung hergestellt wird.

## Revendications

1. Procédé de préparation d'un composé de formule (I)

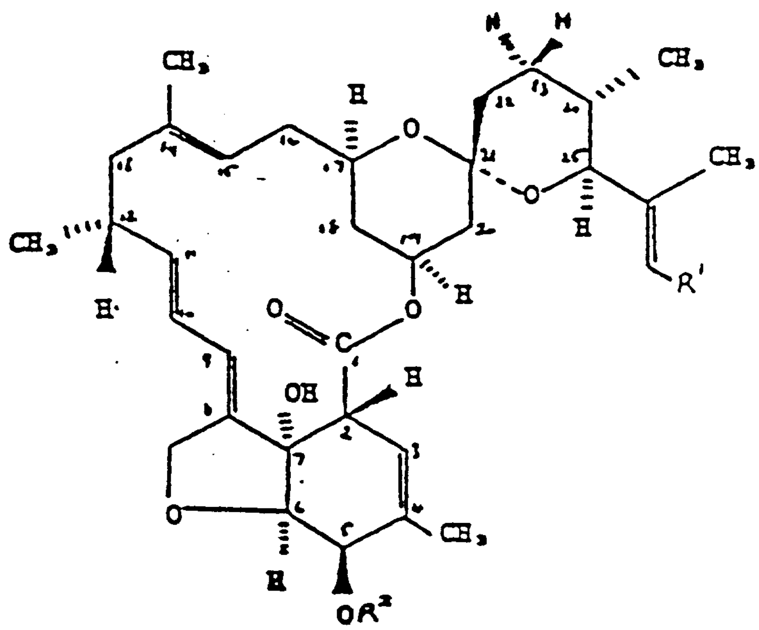

(I)

(dans laquelle $R^1$ représente un groupe méthyle, éthyle ou isopropyle et $OR^2$ représente un groupe hydroxy, méthoxy ou acétyloxy) qui comprend la réduction d'un composé de formule (II)

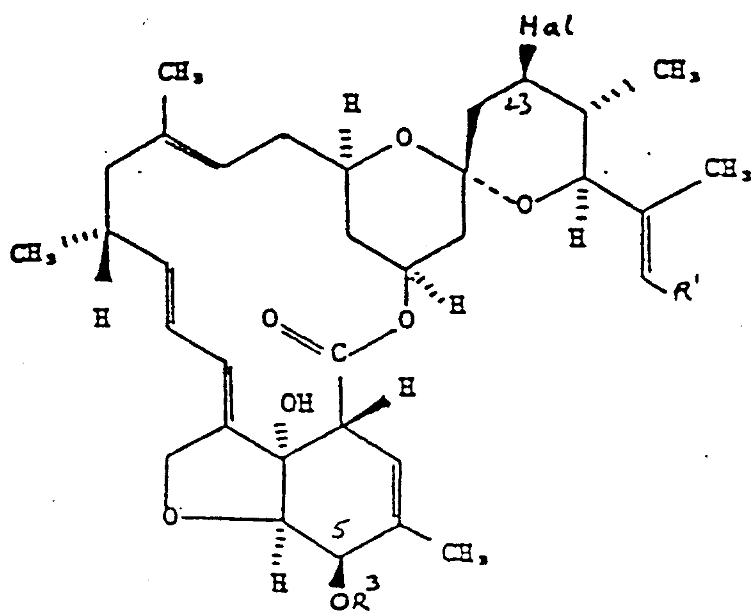

(II)

(dans laquelle $R^1$ est tel que défini ci-dessus, $R^3$ est un atome d'hydrogène, un groupe méthyle ou un groupe protecteur et Hal représente un atome d'halogène) suivie, si on le désire, par une acétylation d'un composé de formule (I) dans laquelle $OR^2$ est un groupe hydroxyle pour former un composé de formule (I) dans laquelle $OR^2$ est un groupe acétyloxy ou par une déprotection pour former un composé 5-hydroxy de formule (I);
à la condition que si $R^3$ est un atome d'hydrogène ou un groupe méthyle, la réduction soit réalisée avec du métal zinc ou un couple zinc-cuivre en présence d'une base ou d'une source protonique.

2. Procédé selon la revendication 1, dans lequel la réduction est réalisée avec du métal zinc ou un couple zinc-cuivre en présence d'une base, de chlorure d'ammonium ou d'acide acétique.

3. Procédé selon la revendication 1, dans lequel le composé de formule (II) est préparé par halogénation du composé 23α-hydroxy correspondant.

4. Procédé selon la revendication 3, dans lequel un composé bromo de formule (II) est préparé par bromation du composé 23α-hydroxy correspondant avec de la triphénylphosphine et du brome dans le diméthylformamide; du tribromure phosphoreux dans le diméthylformamide; du bromure de triméthylsilyl; ou du bromure de lithium et du chlorure de triméthylsilyl.

5. Procédé selon la revendication 1, dans lequel le composé de formule (II) est préparé par réduction du composé 5-céto correspondant.

6. Procédé selon la revendication 1, dans lequel le composé de formule (II) est préparé par réduction du composé 5-céto correspondant avec un borohydrure de métal alkalin ou un hydrure alkoxyaluminium de lithium.

7. Procédé selon la revendication 5, dans lequel ledit composé 5-céto est préparé par halogénation du composé 5-céto 23α-hydroxy correspondant.